# EUROPEAN PATENT APPLICATION

(11) **EP 2 351 533 A1**
(43) Date of publication of application: **03.08.2011**
(21) Application number: 11003057.4
(22) Date of filing: 16.10.2008
(51) Int. Cl.: A61B 17/34

(54) **Access assembly with seal lubricant mechanism**

(30) Priority: 17.10.2007 US 980521 P; 08.10.2008 US 247303
(62) Divisional of application: 08253345.6
(71) Applicant: Tyco Healthcare Group LP, New Haven, CT 06511 (US)
(72) Inventor: Bettuchi, Michael, Middletown, CT 06457 (US); Paul, Stephen R., East Hartford, CT 06118 (US); Heinrich, Russell, Madison, CT 06443 (US); Gresham, Richard D., Guilford, CT 06437 (US); Schiretz, Frank Richard Jr., Middletown, CT 06457 (US); Switzer, Michael D., Highland Park, IL 60035 (US)
(74) Representative: Lloyd, John Scott

(57) **Abstract**

A surgical access apparatus 100 comprising: a housing 110 having at least one vessel 210 associated therewith, the at least one vessel being defined by a plurality of walls 212 that describe an interior space 214 that is configured and dimensioned to retain a fluid, the at least one vessel 210 having at least one egress 216 that is configured and dimensioned for fluid communication such that the fluid may be dispensed from the at least one vessel 210, and a biasing member 218 disposed within the at least one vessel 210 to facilitate dispensation of the fluid, the biasing member 218 including a pusher element 220 that is operatively connected to a spring 222; a cannula sleeve 130 extending from the housing; a seal member 120 disposed within the housing and defining an aperture therethrough; and characterised in that the seal member 120 is movable from a first position to a second position upon the insertion of a surgical instrument into the aperture thereof, the seal member substantially abutting the at least one egress 216 in the first position and at least partially exposing the at least one egress in the second position.

## Description

### CROSS REFERENCE TO RELATED APPLICATIONS

This application claims the benefit of and priority to U.S. Provisional Application Serial No. 60/980,521 filed on October 17, 2007, entitled "ACCESS ASSEMBLY WITH SEAL LUBRICANT MECHANISM", the entire contents of which are hereby incorporated herein by reference.

### BACKGROUND

### 1. Technical Field

The present disclosure relates to surgical devices and, more particularly, to a surgical access assembly for use during minimally invasive surgical procedures.

### 2. Background of the Related Art

Minimally invasive surgical procedures, which include both endoscopic and laparoscopic procedures, permit surgery to be performed on organs, vessels, or the like that are far removed from an opening in the skin. Such procedures are typically performed through a surgical access assembly employing one or more narrow tubes or cannulas inserted percutaneously into the patient.

To better access the underlying organs, etc., typically, the surgical area is insufflated using one or more biocompatible gases. The insufflation gases lift tissue away from the target site such that that a larger, more accessible workspace is created. Accordingly, it is of substantial importance to maintain the integrity of the insufflated environment so as to provide continued access to the surgical site through the duration of the procedure. To this end, surgical access assemblies generally include a seal assembly, which includes a seal or valve member.

Surgical seals generally define an aperture that is dimensioned to receive any surgical instrumentation that might be used during the course of the procedure, and are generally formed of a material that is capable of resilient deformation. The seal's aperture typically defines a diameter that is substantially smaller than that of any surgical instrument to be inserted therethrough such that the aperture is forcibly enlarged by the instrument, and the resilient nature of the material comprising the seal allows it to stretch in order to accommodate the surgical instrument. This stretching creates a force that is applied to the instrument and results in the formation of a substantially fluid tight seal therewith, substantially preventing the escape of insufflation gases through the cannula of the access assembly. Consequently, however, it is often necessary for a clinician to apply significant pressure in an effort to move the surgical instrument longitudinally through the seal.

While it is known in the art that lubricating either the surgical instrument or the seal member may reduce the force necessary to move the surgical instrument longitudinally distally during the course of a procedure, there exists a continuing need for surgical assemblies and seal members that incorporate lubricous elements.

### SUMMARY

In one aspect of the present disclosure, a surgical access apparatus is disclosed that includes a housing with a seal member disposed therein. The seal member includes at least one reservoir adapted to accommodate a fluid and having at least one egress such that the fluid may be discharged from the at least one reservoir. The at least one egress is configured and dimensioned to communicate the at least one fluid externally of the seal member.

In one embodiment, the at least one reservoir includes a plurality of reservoirs that may be substantially arcuate in configuration and concentrically disposed within the seal member.

The seal member may be at least partially formed from a deformable material such that the seal member may transition from a first condition, in which the fluid is retained within the at least one reservoir, to a second condition, in which the fluid is discharged from the at least one reservoir. In the first condition, the at least one reservoir defines a fluid retaining capacity that is decreased as the seal member transitions from the first condition to the second condition, thereby causing the expulsion of fluid from the at least one reservoir.

In one embodiment, the seal member includes a veneer member secured to the periphery thereof for facilitating the retention of the fluid within the at least one reservoir. The veneer member is adapted to be penetrated by a surgical instrument upon its insertion into the housing.

In another aspect of the present disclosure, a surgical access apparatus is disclosed which comprises a housing having at least one vessel associated therewith, a cannula sleeve extending from the housing, and a seal member disposed within the housing and defining an aperture therethrough. The at least one vessel is defined by a plurality of walls that describe an interior space that is configured and dimensioned to retain a fluid. The at least one vessel includes at least one egress that is configured and dimensioned for fluid communication such that the fluid may be dispensed from the at least one vessel.

The at least one vessel may be mounted to an inner wall of the housing and may be engagable with a surgical instrument upon its insertion into the surgical access apparatus such that the fluid retained within the at least one vessel may be discharged therefrom through the at least one egress.

In one embodiment, the housing defines at least one channel therein that is configured and dimensioned to communicate a gas. The channel is disposed distally of the at least one egress such that the fluid may be drawn out of the at least one vessel upon the communication of the gas.

In another embodiment, the surgical access apparatus includes at least one pump member operatively associated with the at least one vessel.

In yet another embodiment, the surgical access apparatus further includes a latch member operatively associated with the at least one pump member and disposed on the inner wall of the housing. The latch member is movable from a first position to a second position upon the insertion of a surgical instrument into the housing such that the at least one pump member may be activated.

The seal member may be movable from a first position to a second position upon the insertion of a surgical instrument into the aperture thereof. In the first position, the seal member substantially abuts the at least one egress, and in the second position, the at least one egress is at least partially exposed.

The surgical access apparatus may further include a biasing member disposed within the at least one vessel that facilitates dispensation of the fluid retained therein.

In an alternate embodiment, the at least one vessel also includes at least one ingress disposed distally of the at least one egress, and the cannula sleeve defines a channel therethrough. In this embodiment, the at least one ingress in fluid communication with the interior space defined by the plurality of walls of the at least one vessel through a channel defined in the cannula sleeve.

In still another embodiment, the surgical access apparatus further includes a one-way valve disposed within the channel adjacent the at least one ingress. The one-way valve is configured and dimensioned such that the fluid retained within the at least one reservoir is substantially prevented from exiting the channel through the at least one ingress.

In another aspect of the present disclosure, a surgical access apparatus is disclosed that includes a housing having at least one reservoir disposed therein that is adapted to retain a fluid, a seal member disposed within the housing, and a wick member in fluid communication with the at least one reservoir, wherein the wick member is at least partially disposed within the housing and proximally of the seal member.

In yet another aspect of the present disclosure, a surgical access apparatus is disclosed which includes a housing having a seal member and a bladder member disposed therein. The bladder member defines an internal cavity that is adapted to retain a fluid therein that is exuded from the bladder member upon the puncture thereof by a surgical instrument.

In a final aspect of the present disclosure, a surgical access apparatus is disclosed that includes a housing having a seal member disposed therein and a grommet member associated therewith. The grommet member is adapted for penetration by a needle member that is configured and dimensioned to communicate a fluid therethrough. The grommet member is oriented such that the fluid may be communicated through the needle member and about the seal member.

These and other features of the valve disclosed herein will become more readily apparent to those skilled in the art from the following detailed description of various embodiments of the present disclosure.

### BRIEF DESCRIPTION OF THE DRAWINGS

Various embodiments of the present disclosure are described hereinbelow with references to the drawings, wherein:

**FIGS. 1-2** are perspective views of a surgical access apparatus in accordance with the principles of the present disclosure;

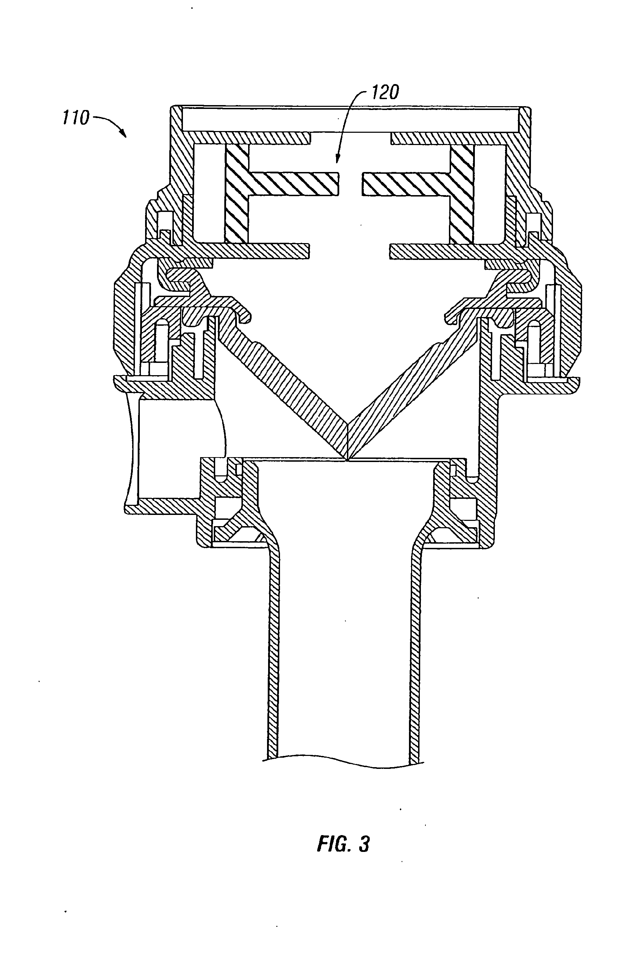

**FIG. 3** is a side cross-sectional view of the surgical access apparatus of **FIGS. 1**-2;

**FIG. 4A** is a side cross-sectional view of one embodiment of the surgical access apparatus of **FIGS. 1-3** depicting a seal member in a first condition that includes at least one reservoir;;

**FIG. 4B** is top plan view of the seal member of **FIG. 4A****;**

**FIG. 4C** is a side cross-sectional view of the surgical access apparatus of **FIG. 4A** depicting the seal member in a second condition with a surgical instrument inserted therethrough;

**FIG. 5A** is a side cross-sectional view of another embodiment of the surgical access apparatus of **FIGS. 1-3** depicting a seal member in a first condition that includes at least one reservoir;

**FIG. 5B** is top plan view of the seal member of **FIG. 5A****;**

**FIG. 5C** is a side cross-sectional view of the surgical access apparatus of **FIG. 5A** depicting the seal member in a second condition with a surgical instrument inserted therethrough;

**FIG. 6A** is a side cross-sectional view of one embodiment of the surgical access apparatus of **FIGS. 1-3** depicting a seal member, which includes at least one reservoir, in a first condition and a veneer member;

**FIG. 6B** is top plan view of the seal member of **FIG. 6A****;**

**FIG. 6C** is a side cross-sectional view of the surgical access apparatus of FIG. 6A depicting the seal member and the veneer member with a surgical instrument inserted therethrough;

**FIG. 7A** is a side cross-sectional view of a surgical access apparatus, including a seal member in a first condition, in accordance with the principles of the present disclosure, wherein the surgical access apparatus includes a housing having at least one vessel associated therewith;

**FIG. 7B** is a side cross-sectional view of the surgical access apparatus of **FIG. 7A** depicting the seal member in a second condition with a surgical instrument inserted therethrough;

**FIG. 8A** is a side cross-sectional view of one embodiment of the surgical access apparatus of **FIG. 7A** depicting a seal member in a first condition, wherein the ;

**FIG. 8B** is a side cross-sectional view of the surgical access apparatus of **FIG. 8A** depicting the seal member in a second condition with a surgical instrument inserted therethrough;

**FIG. 9A** is a side cross-sectional view of one embodiment of the surgical access apparatus of **FIG. 7A** including a sleeve having a channel defined therein and depicting a seal member in a first condition;

**FIG. 9B** is a side cross-sectional view of the surgical access apparatus of **FIG. 8A** depicting the seal member in a second condition with a surgical instrument inserted therethrough;

**FIG. 9C** is a side cross-sectional view of one embodiment of the surgical access apparatus of **FIG. 9A** further including a one-way valve;

**FIG. 10A** is a side cross-sectional view of one embodiment of the surgical access apparatus of **FIG. 7A** including a housing having a channel defined therein and depicting a seal member in a first condition;

**FIG. 10B** is a side cross-sectional view of the surgical access apparatus of **FIG. 10A** depicting the seal member in a second condition with a surgical instrument inserted therethrough;

**FIG. 11A** is a side cross-sectional view of one embodiment of the surgical access apparatus of **FIG. 7A** including a pump member, a latch member, and a seal member, wherein the latch member and the seal member are each in a first condition;

**FIG. 11B** **is** a side cross-sectional view of the surgical access apparatus of **FIG. 11A** wherein the latch member and the seal member are each in a second condition;

**FIG. 12A** is a side cross-sectional view of one embodiment of the surgical access apparatus of **FIG. 7A** wherein the at least one vessel is disposed on an inner wall of the housing;

**FIG. 12B** is a top plan view of the at least one vessel depicted of **FIG. 12A**;

**FIG. 12C** is a side cross sectional view of the at least one vessel of **FIGS. 12A**-**12B;**

**FIG. 12D** is a side cross sectional view of the surgical access apparatus of **FIG**. **12A** depicting the at least one vessel in a second condition;

**FIG. 13A** is a side cross-sectional view of another surgical access apparatus, in accordance with the principles of the present disclosure, including a wick member and a seal member, wherein the seal member is in a first condition;

**FIG. 13B** is a side cross sectional view of the surgical access apparatus of **FIG**. **13A** depicting the seal member in a second condition;

**FIG. 14A** is a side cross-sectional view of yet another surgical access apparatus, in accordance with the principles of the present disclosure, including a seal member and a bladder member, wherein the seal member is in a first condition;

**FIG. 14B** is a side cross-sectional view of the surgical access apparatus of **FIG. 14A** depicting the seal member and the bladder member with a surgical instrument inserted therethrough;

**FIG. 15A** is a side cross-sectional view of still another surgical access apparatus, in accordance with the principles of the present disclosure, including a seal member and a grommet member, wherein the seal member is in a first condition; and

**FIG. 15B** is a side cross-sectional view of the surgical access apparatus of **FIG. 15A** depicting the seal member in a second condition and a needle member inserted thorough the grommet member.

### DETAILED DESCRIPTION OF THE EMBODIMENTS

In the drawings and in the description which follows, in which like reference numerals identify similar or identical elements, the term "proximal" will refer to the end of the apparatus which is closest to the clinician, while the term "distal" will refer to the end which is furthest from the clinician, as is traditional and known in the art.

Throughout the present disclosure, the term "seal" or "seal member" should be understood as referring to any seal or valve member, formed of any suitable biocompatible material that is at least semi-resilient in nature and capable of deformation, which may be used in connection with any surgical portal or access assembly, apparatus, or device.

In each of the embodiments described hereinbelow, the term "fluid" should be understood as referring to any biocompatible substance or fluid that is at least semi-lubricous in nature. In addition, it should be understood that the "fluid" may refer to either a single substance or fluid, or to a combination of a plurality of substances or fluids, which may or may not have medicinal or therapeutic characteristics.

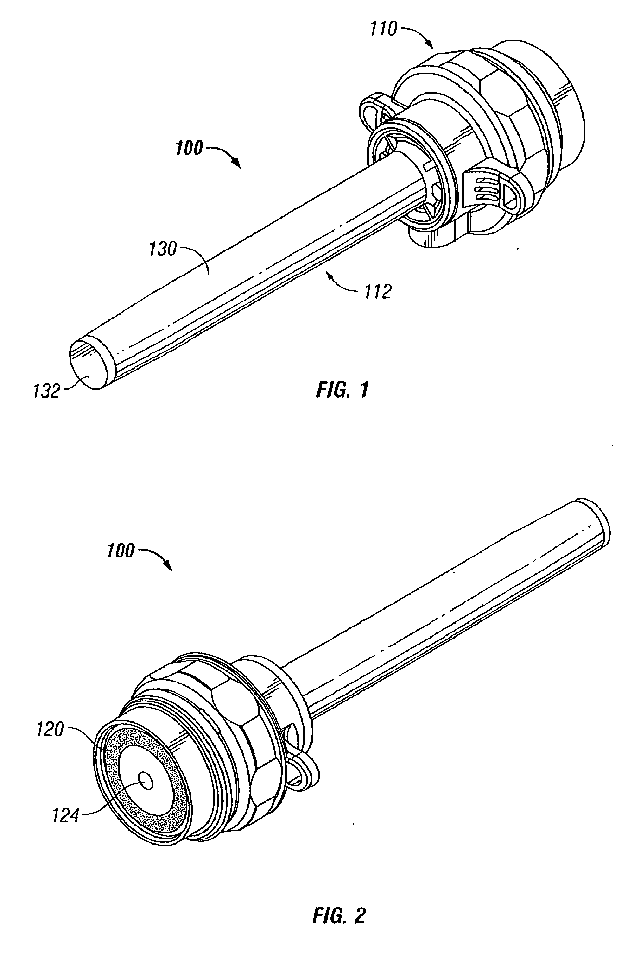

Referring now to the drawings, in which like reference numerals identify identical or substantially similar parts throughout the several views, **FIGS. 1-2** illustrate a surgical access apparatus 100 in general accordance with the principles of the present disclosure. Surgical access apparatus 100 includes a housing 110 having a seal member 120 disposed therein that defines an aperture 124, and a cannula **112** extending distally therefrom having a sleeve 130. Sleeve 130 defines a lumen or passageway 132 that is configured and dimensioned for the reception of a surgical instrument (not shown).

Throughout the present disclosure and in the figures, the surgical apparatus will be depicted as including elements that are typically associated with a cannula assembly, e.g. housing 110 and sleeve 130. It should be understood, however, that the principles of the present disclosure are applicable to any surgical access or portal apparatus or device suitable for the intended purpose of facilitating access to a patient's internal cavities, organs, tissues, or during the course of a minimally invasive procedure.

Each apparatus disclosed herein is adapted for use with an obturator assembly (not shown). The obturator assembly may include an obturator, trocar, or similar penetrating device having a tip that may be blunt, or incisive, appreciably transparent or opaque, retractable or fixed, or any other such variation, either currently known or later devised. The obturator assembly is typically utilized to penetrate tissue such that sleeve **130** of apparatus **100** may be percutaneously introduced into a patient. Subsequently, the obturator assembly is removed from the apparatus **100** to permit the introduction of one or more surgical instruments through passage **132** in sleeve **130.**

Each apparatus disclosed herein may be formed, either in whole or in part, of any suitable medical grade material, such as stainless steel, polymeric materials, or the like that may be partially or completely transparent or opaque. Generally, sleeve **130** will have a diameter within the range of about 4.5 mm to about 15 mm, although a sleeve having a substantially larger, or smaller, diameter is within the scope of the present disclosure.

As seen in **FIG. 2****,** housing **110** is configured and dimensioned to receive seal member **120,** which may be either fixedly or removably disposed therein, in any suitable manner. Further details regarding housing **110** may be obtained through reference to commonly assigned U.S. Patent Application Publication No. 2006/0149305to Cuevas *el al.,* which was published on July 6^{th}, 2006 and is incorporated herein by reference in its entirety.

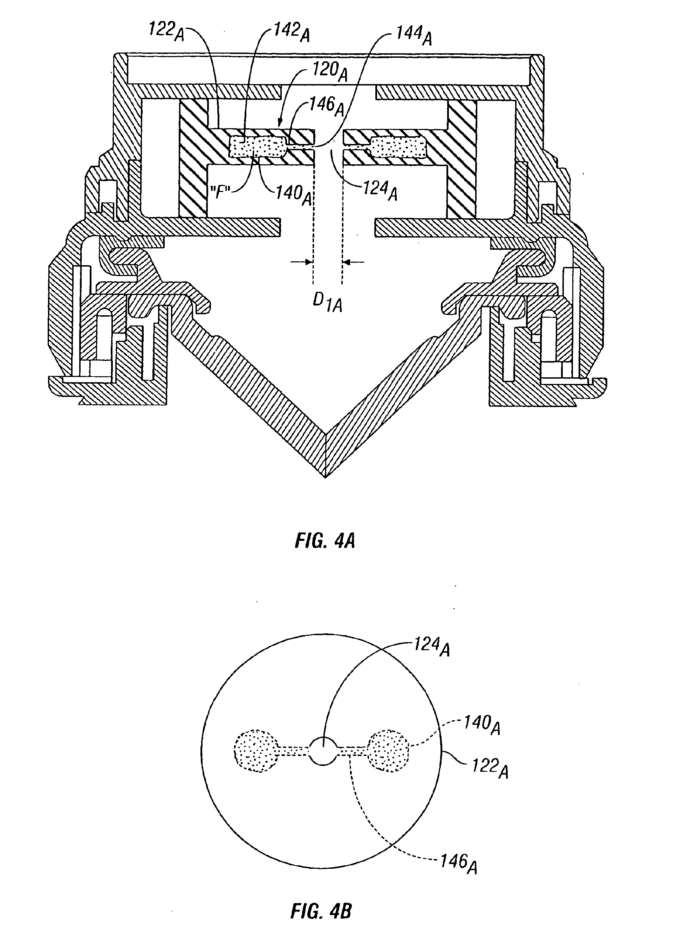

Referring now to **FIGS. 4A-6C****,** various embodiments of seal member **120** will be discussed. In particular, with reference to **FIGS. 4A-4C****,** seal member **120_{A}** includes an outer wall **122_{A}** and at least one reservoir **140_{A}** that defines an interior space **142_{A}** that is configured and dimensioned to accommodate a fluid or substance **"F"**. Although depicted as substantially irregular in configuration, reservoir **140A** may exhibit any configuration suitable for the intended purpose of accommodating fluid "**F**", including but not being limited to, a circular or elliptical configuration. In one embodiment, seal member **120_{A}** may include four reservoirs (not shown) that are substantially identical to and disposed equidistant from one another within an outer wall **122_{A}** of seal member **120_{A}**. Reservoir **140_{A}** includes at least one egress **144_{A}** formed in outer wall **122_{A}** that is configured and dimensioned to permit fluid **"F"** to be discharged therefrom. Egress **144_{A}** may be any opening, channel, aperture, breach, or the like facilitating the communication of a fluid. Egress **144_{A}** is in fluid communication with the interior space **142_{A}** of reservoir **140_{A}** through a channel **146** that is configured and dimensioned to communicate a fluid.

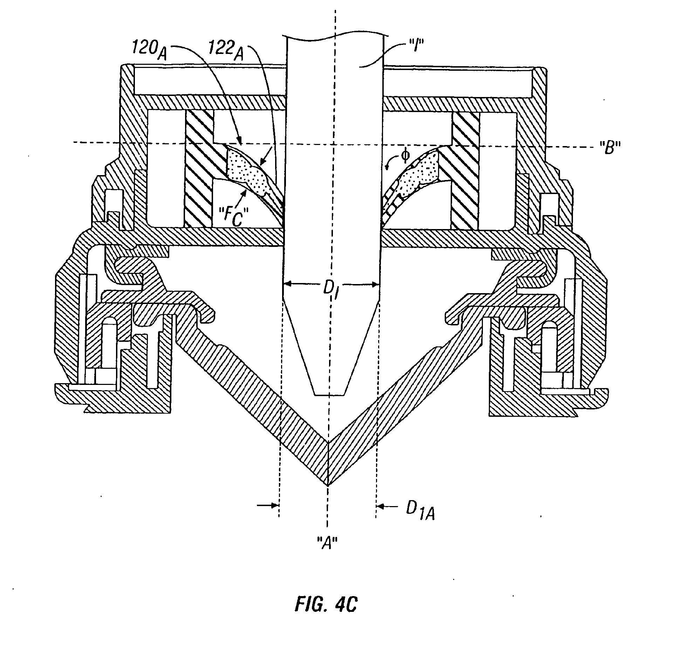

Aperture **124_{A}** of seal member **120_{A}** is configured and dimensioned to receive a surgical instrument **"I"** defining a longitudinal axis **"A".** Prior to insertion, seal member **120_{A}** is in a first, or initial, condition **(****FIGS. 4A-4B****)**, in which aperture **124_{A}** defines a first, or initial, diameter "**D_{IA}**" that is lesser than the diameter "**D_{I}**" of instrument **"I" (****FIG. 4C****).** Upon the insertion of instrument "**I**", seal member **120_{A}** begins to transition into a second condition (FIG. 4C) in which aperture **124_{A}** defines a second, or enlarged diameter, "**D_{2A}**" that substantially approximates the diameter "**D_{I}**" of instrument "**I**". In the second condition, seal member **120_{A}** is deflected in a downward, or distal, direction such that seal member **120_{A}** defines an angle **θ** formed with an axis **"B"** that is transverse to the longitudinal axis **"A"** of instrument **"I"**. As aperture **124_{A}** enlarges, seal member **120_{A}** experiences a compressive force "**F_{C}**" that is directed radially inward. Force "**F_{C}**" is transmitted to reservoir **140_{A}** through the material comprising seal member **120_{A}** and thereby deforms reservoir **140_{A}**, decreasing the volume of interior space **142_{A}**. This decrease in volume causes fluid **"F"** to be discharged from reservoir **140_{A}**, through channel **146,** and ultimately through egress **144_{A}** onto outer wall **122_{A}** of seal member **120_{A}**. After being discharged or dispensed onto outer wall **122_{A}**, fluid "**F**" traverses outer wall **122_{A}** of seal member **120_{A}** and approaches aperture **124_{A}**, being assisted by the force of gravity acting thereupon in the direction of arrow **"G"**, given the angle of deflection **θ** of seal member **120_{A}** formed with transverse axis **"B"** in the second condition. When fluid **"F"** reaches aperture **124_{A,}** it contacts, and thereby lubricates instrument **"I"**, facilitating the longitudinal manipulation of instrument **"I"** within seal member **120_{A}**.

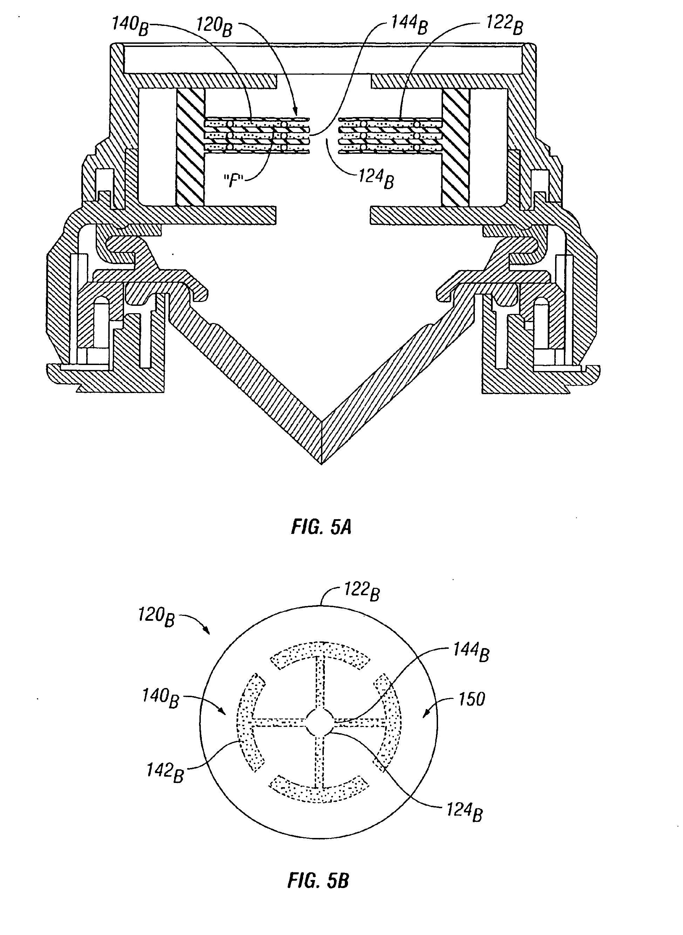

As seen in **FIGS. 5A-5C**, in an alternate embodiment, seal member **120_{B}** includes reservoirs **140₈** are substantially arcuate in configuration. Reservoirs **140_{B}** may be arranged in one or more concentric rings **150** within outer wall **122_{B}** of seal member **120_{B}**. While seal member **120_{B}** is depicted as incorporating a plurality of arcuate reservoirs, a seal member including a single arcuate reservoir is also within the scope of the present disclosure.

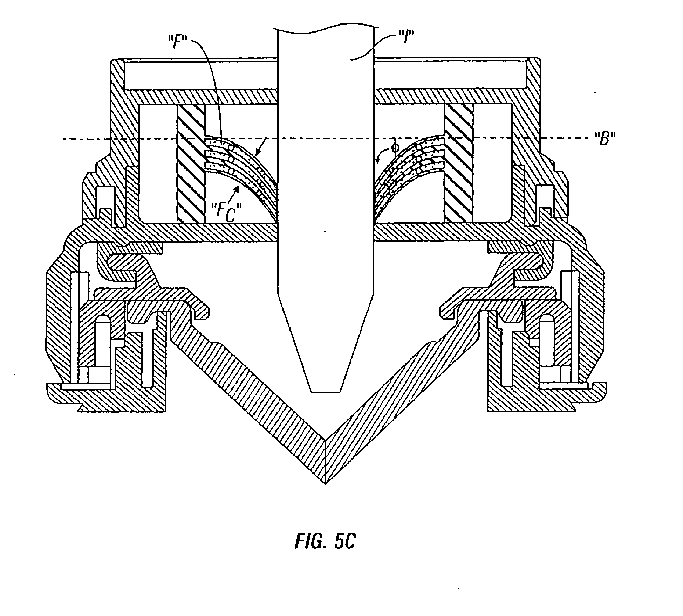

Upon the introduction of instrument **"I"** to seal member **120_{B}**, seal member **120_{B}** transitions from the first condition (**FIGS. 5A-5B**) to the second condition (**FIG. 5C**). During this transition, aperture **124_{B}** enlarges and deforms, thereby creating the compressive force **"Fc"** that acts upon reservoirs **140_{B}** and decreases the volume of interior space **142_{B}** such that fluid **"F"** is discharged therefrom through channels **146_{B}** and egresses **144_{B}**. In addition, during the transition from the first condition to the second condition, seal member **120_{B}** is deflected distally at angle **θ** formed with transverse axis **"B"**. This downward or distal deflection contributes to the communication of fluid **"F"** toward aperture **124_{B}** and instrument "**I**", as discussed above with respect to the embodiment of **FIGS. 4A-4C****.**

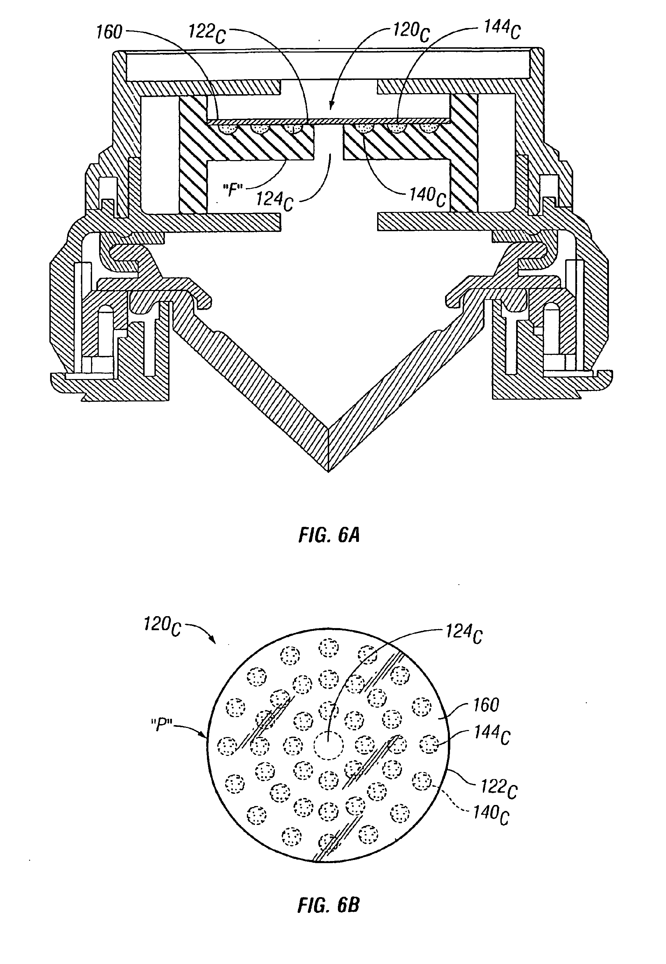

Referring now to **FIGS. 6A-6C**, in another embodiment, seal member **120_{C}** includes reservoirs **140_{C}**. Reservoirs **140_{C}** constitute concave formations in the outer wall **122_{C}** of seal member **120_{C}**. As may be appreciated through reference to **FIGS. 6A-6C**, in this embodiment, there is no need for a channel facilitating the communication of fluid between egress **144_{C}** and reservoir **140_{C}**, as egress **144_{C}** is inherently defined by the concave configuration of reservoir **140_{C}**. Seal member **120_{C}** further includes a veneer member **160** that is secured to the periphery "**P**" of seal member **120_{C}.** Veneer member 160 may be formed of any material suitable for the intended purpose of maintaining the disposition of fluid **"F"** within reservoirs **140_{C}** prior to the introduction of instrument "**I**". Upon introduction, instrument "**I"** punctures or penetrates veneer member **160** such that instrument **"I"** may pass therethrough and into aperture **124_{C}** of seal member **120_{C}**. Alternatively, veneer member **160** may be removed from seal member **120_{C}** prior to the introduction of instrument **"I".**

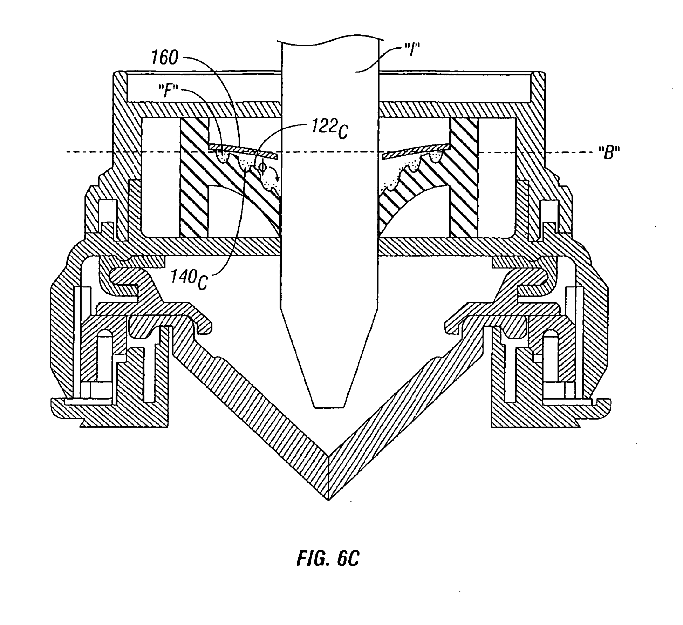

Upon the introduction of instrument **"I",** seal member **120_{C}** transitions from a first condition (**FIGS. 6A-6B**) to a second condition **(****FIG. 6C****).** During this transition, seal member **120_{C}** deforms such that the volume of reservoirs **140_{C}** are decreased. This decrease in volume of reservoirs **140_{C}** discharges fluid **"F"** therefrom, as discussed above with respect to the embodiments of **FIGS. 4A-5C**. In addition, during the transition from the first condition to the second condition, seal member **120_{C}** is deflected distally at angle **θ** formed with transverse axis **"B".** This downward or distal deflection contributes to the communication of fluid **"F"** across outer wall **122_{C}** of seal member **120_{C}** toward aperture **124_{C}** and instrument **"I",** as discussed above with respect to the embodiments of **FIGS. 4A-5C****.**

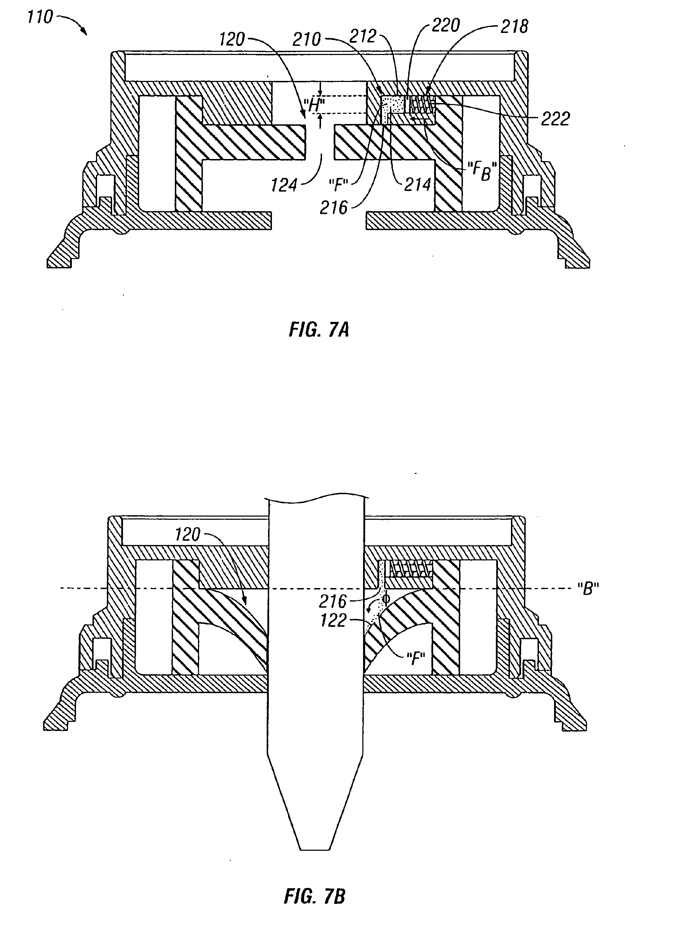

Referring now to **FIGS. 7A-12C****,** an alternate aspect of the present disclosure will be discussed in which a surgical access apparatus is disclosed that includes a housing **110**, a sleeve **130 (****FIG. 1****)**, and seal member **120** defining an aperture **124** therethrough

Referring in particular to **FIGS. 7A-7B**, in one embodiment, housing **110** includes at least one vessel **210** associated therewith. Vessel **210** is defined by a plurality of walls **212** that describe an interior space **214** that is configured and dimensioned to retain fluid **"F"**. Vessel **210** includes at least one egress **216** that is disposed proximally of seal member **120.** Egress **216** is configured and dimensioned for fluid communication such that the fluid **"F"** may be dispensed from the at least one vessel **210**. Vessel **210** further includes a biasing member **218** that is disposed within interior space **214**. As shown, biasing member **218** includes a pusher element **220** that is operatively connected to a spring **222.** Pusher element **220** defines a height **"H"** that approximates that of interior space **214** such that pusher element **220** may advance fluid **"F"** through vessel **210** under the influence of a biasing force "**F_{B}**" created by spring **222,** as discussed in further detail below. Biasing member **218** may be any member suitable for the intended purpose of creating a biasing force "**F_{B}**" sufficient in magnitude to advance fluid **"F"** through vessel **210.**

Prior to the introduction of a surgical instrument thereto, seal member **120** is in a first, or initial, condition **(****FIG. 7A****).** In this first condition, seal member **120** substantially abuts egress **216**, thereby obstructing, and substantially preventing the flow of fluid **"F"** from vessel **210** therethrough. Additionally, the presence of fluid **"F"** in vessel **210** in the first condition deforms or compresses spring **222,** and consequently pusher element **220,** such that biasing force "**F_{B}**" is created and stored as potential energy in spring **222**. Upon the introduction of surgical instrument **"I",** seal member **120** transitions from the first condition to a second condition **(****FIG. 7B****).** During this transition, aperture **124** of seal member **120** is enlarged such that a substantially fluid tight seal is formed between seal member **120** and instrument **"I".** Additionally, during the transition from the first condition to the second condition, seal member **120** is deflected distally at angle **θ** formed with transverse axis **"B"**, as discussed above with respect to the embodiments of **FIGS. 4A-6C****.** In the second condition, seal member **120** is displaced from egress **216** such that the flow of fluid **"F"** is no longer obstructed and the communication of fluid **"F"** from vessel **210** through egress **216** is permitted.

The communication of fluid **"F"** from vessel **210** through egress **216** is facilitated not only by the gravitational force acting upon fluid "**F**", but by the biasing force "**F_{B}**" exerted upon fluid **"F"** by biasing member **218**. In the first condition, biasing force "**F_{B}**" endeavors to expel fluid **"F"** from vessel **210.** However, biasing force "**F_{B}**" is insufficient in magnitude to displace seal member **120** distally, and thereby expose egress **216** and permit the communication of fluid **"F"** therethrough. Accordingly, in the first position, biasing force "**F_{B}**" acts only to pressurize fluid **"F"**. In the second condition, however, as seal member x is displaced distally by instrument **"I"** and egress **216** is exposed, the flow of fluid "**F**" therethrough is no longer obstructed, as discussed above, and biasing force "**F_{B}**", or the potential energy stored in spring **222,** is free to advance pusher element **220** such that fluid **"F"** is forced from vessel **210.**

As seen in **FIGS. 7A-7B** and discussed above, egress **216** is disposed proximally of seal member **120.** Accordingly, as fluid **"F"** is discharged from vessel **210** through egress **216**, it is dispensed onto outer wall **122** of seal member **120.** Thereafter, fluid **"F"** is inwardly communicated across outer wall **122** of seal member **120**, e.g., towards instrument **"I"** given the distal deflection of seal member **120** at angle **θ** formed with transverse axis **"B"** in the second condition. When fluid **"F"** reaches aperture **124** of seal member **120,** it contacts, and thereby lubricates instrument "**I**", facilitating the longitudinal manipulation of instrument "**I**" within seal member **120,** as discussed above with respect to the embodiments of **FIGS. 4A-6C**.

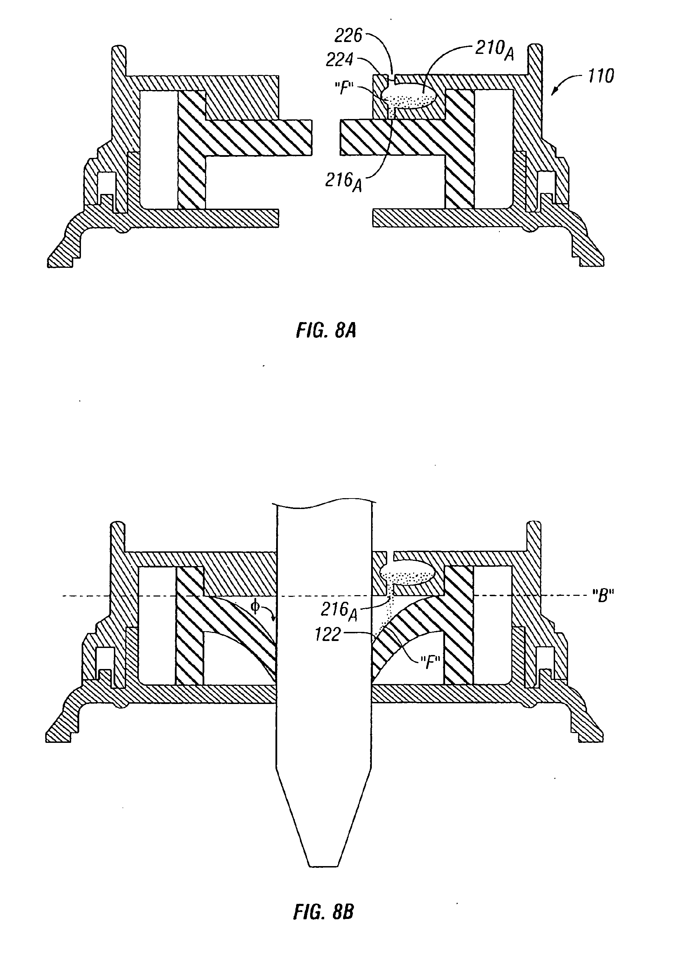

As seen in **FIGS. 8A-8B**, vessel **210_{A}** includes a channel **224** that extends proximally therefrom and terminates in an opening **226** in housing **110.** Opening **226** and channel **224** are configured and dimensioned to facilitate the flow of ambient air into and through vessel **210_{A}** and egress **216_{A}.**

In this embodiment, when seal member **120** is in the first condition **(****FIG. 8A****),** atmospheric pressure is applied to fluid **"F"** through opening **226** and channel **224** in housing **110.** This pressure endeavors to force fluid **"F"** from vessel **210_{A}** through egress **216_{A}**. However, fluid **"F"** is substantially preventing from exiting vessel **210_{A}** through egress **216_{A}** given the abutment thereof with seal member **120** in the first condition. Accordingly, in the first position, the atmospheric pressure applied to fluid **"F"** through opening **226** and channel **224** formed in housing **110** serves only to pressurize fluid "**F**", as discussed above with respect to the previous embodiment. As seal member **120** transitions from the first condition to the second condition **(****FIG. 8B****),** the pressurized fluid **"F"** is discharged from vessel **210_{A}** through egress **216_{A}** and onto an outer wall **122** of seal member **120**. Subsequently, fluid "**F**" is communicated inwardly, across outer wall **122** of seal member **120,** ultimately contacting, and thereby lubricating instrument "**I**", as discussed above with respect to the previous embodiments.

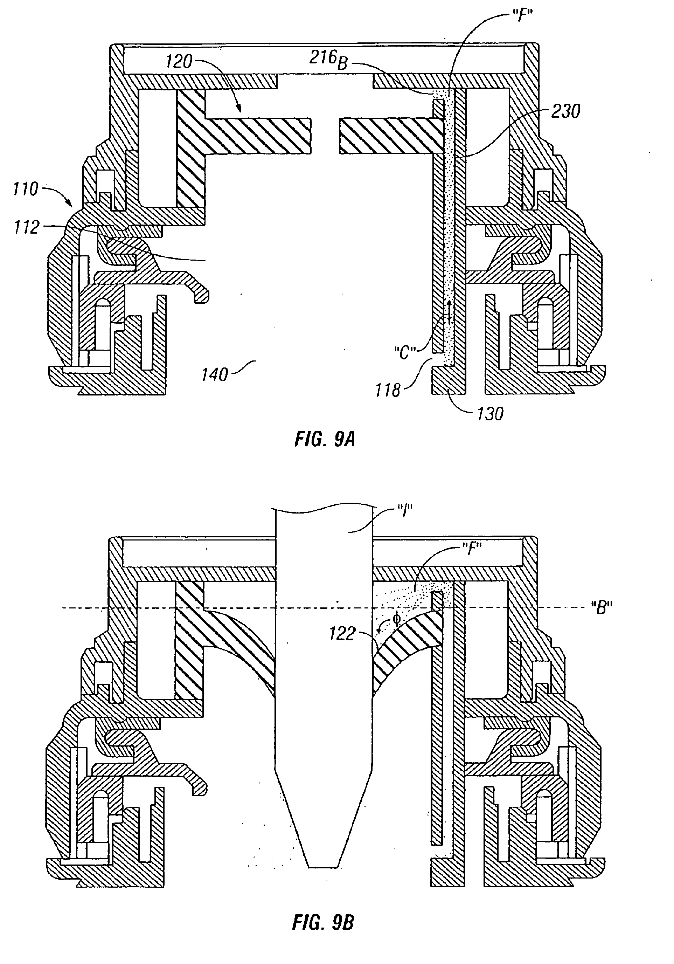

Referring to **FIGS. 9A-9C**, in yet another embodiment, vessel **210_{B}** includes at least one ingress **228**. Ingress **228** is disposed distally of egress **216_{B}** at a distal end of a channel 230 formed in cannula sleeve **130.** Channel **130** and ingress **228** are each configured and dimensioned to communicate a fluid, e.g. an insulation gas **140**, such that fluid communication may be established between cannula **112** and the interior space **214_{B}** of vessel **210_{B}**.

In this embodiment, prior to the transition of seal member **120** from the first condition **(****FIG. 9A****)** to the second condition **(****FIG. 9B****),** pressurized insufflation gas **140** is pumped into cannula **112** through an insufflation port (not shown) formed either in housing **110** or sleeve **130,** as it is known in the art. As cannula **112** fills with insufflation gas **140,** the pressurized gas **140** is forced through ingress **228** into channel **230** and is subsequently communicated proximally, in the direction of arrows **"C"**, to vessel **210_{B}** where fluid **"F"** is retained. The communication of gas **140** into vessel **210_{B}** forces fluid "F" therefrom through egress **216_{B}** and onto the outer wall **122** of seal member **120.**

As seal member **120** transitions from the first condition to the second condition upon the introduction of instrument "**I**", seal member **120** is deflected distally at angle **θ** formed with transverse axis **"B",** such that fluid **"F"** is communicated inwardly, across outer wall **122** of seal member **120**, ultimately contacting, and thereby lubricating instrument **"I"**, as discussed above with respect to the embodiments of **FIGS. 4A-8B****.**

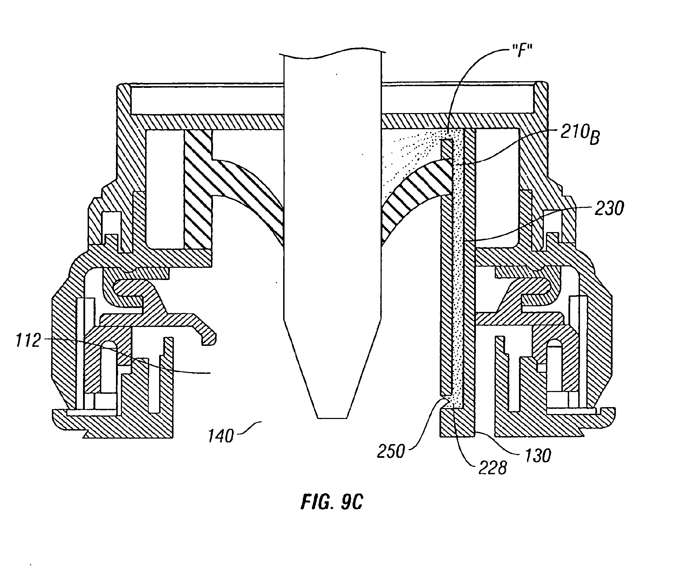

As seen in **FIG. 9C****,** a one-way valve **250** may be disposed within the channel **230** formed in sleeve **130.** One-way valve **250** may be any valve or member suitable for the intended purpose of permitting the flow of insufflation gas **140** from cannula **112** into channel **230** while substantially prohibiting the distal flow of fluid **"F",** if any, from vessel **210_{B}** into cannula **112** through channel **230** and ingress **228.** As shown, one-way valve **250** is disposed substantially adjacent ingress **228**. However, it is contemplated that one-way valve **250** may be disposed at any location suitable for its intended purpose.

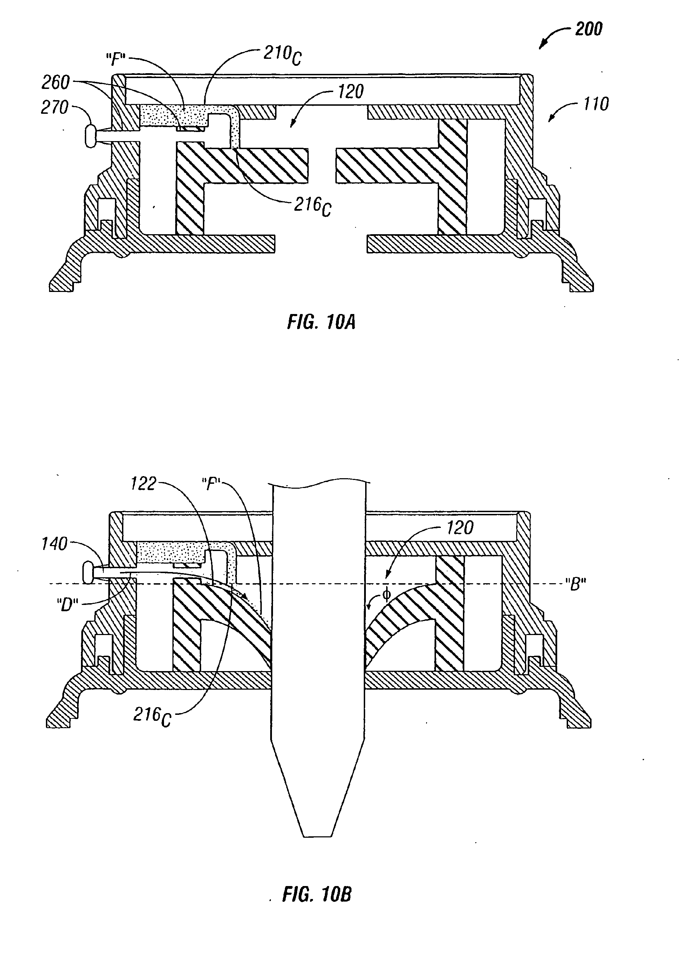

With respect to **FIGS. 10A-10B**, in an alternate embodiment of apparatus **200,** housing **110** includes a conduit **260** defined therein that is in fluid communication with an inlet port **270** formed in housing **110.** Conduit **260** extends through seal member **120** and is disposed distally of egress **216_{C}** formed in vessel **210_{C}** and. Conduit **260** and is configured and dimensioned to communicate a fluid, e.g. insufflation gas **140**, therethrough.

When seal member **120** is in the first condition (**FIG. 10A**), seal member **120** substantially prevents the escapes of fluid "**F**" from vessel **210_{C}** by obscuring egress **216_{C},** as discussed above with respect to the embodiments of **FIGS. 7A-9C**. As seal member **120** transitions from the first condition to the second condition (**FIG. 10B**) upon the introduction of surgical instrument **"I",** seal member **120** is deflected distally at angle **θ** formed with transverse axis **"B"** such that egress **216_{C}** is no longer obscured. Concurrently, gas **140** is pumped into cannula **112** through port **270** in the direction of arrow **"D"** and is communicated through conduit **260** such that it flows past egress **216_{C}**. As gas **140** passes egress **216_{C}**, it begins to draw or pull fluid **"F"** from vessel **210_{C}** through egress **216_{C}**. Subsequently, fluid **"F"** exits vessel **210_{C}** through egress **216c**, under the influence of both gravity and the flow of gas **140** past egress **216_{C}**, and is discharged onto outer wall **122** of seal member **120**. Thereafter, fluid **"F"** is communicated inwardly, across outer wall **122** of seal member **120,** ultimately contacting, and thereby lubricating instrument "**I**", as discussed above with respect to each of the embodiments discussed above.

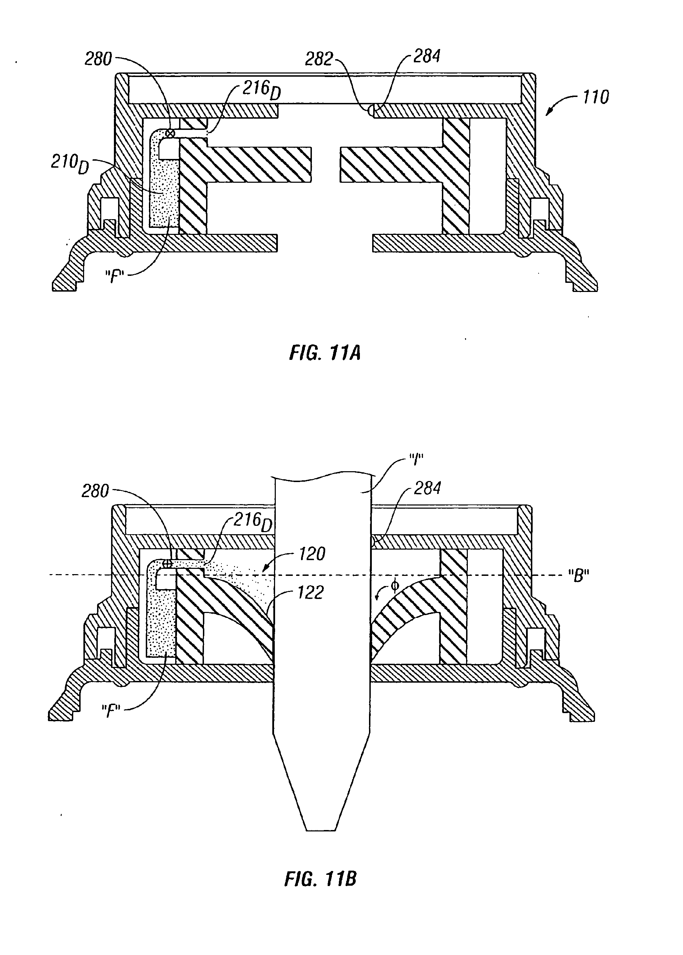

Referring now to **FIGS. 11A-11B**, in another embodiment, one or more pump members **280** are operatively associated with vessel **210_{D}** and a latch member **282**. Pump member, or members, **280** may be any mechanism suitable for the intended purpose of facilitating the discharge of fluid **"F"** from vessel **210_{D}** through egress **216_{D}**.

Latch member **282** is disposed proximally of seal member **120** on an inner wall **284** of housing **110** at any location that facilitates the engagement of latch member **282** and surgical instrument **"I"** upon the insertion thereof into housing **110**, as discussed in further detail below. Latch member **282** is configured and dimensioned for movement between a first position (**FIG. 11A**) and a second position (**FIG. 11B**). In the first position, latch member **282** is configured and dimensioned to engage surgical instrument **"I"** in any suitable manner. As shown, in one embodiment, in the first position, latch member **282** extends radially inward, i.e. into housing **110,** such that latch member **282** may contact instrument **"I"** upon the insertion thereof, and in the second position, latch member **282** is displaced radially outward.

Latch member **282** is operatively associated with a biasing mechanism (not shown), e.g. a spring, that maintains latch member **282** in the first position. Upon the displacement of latch member **282** by instrument **"I"**, a biasing force is created in the biasing mechanism (not shown) that is directed radially inward, thereby returning the latch member **282** to the first position upon the removal of instrument **"I".**

In the second position, latch member **282** is configured and dimensioned to activate pump **280.** Latch member **282** may activate pump **280** in any suitable manner, including but not limited to, completing an electrical circuit when in the second position such that energy may be delivered to pump **280** from a suitable energy source (not shown), such as a battery or a generator. The activation of pump **280** causes the continuous discharge of fluid "**F**" from vessel **210_{D}** through egress **216_{D}** and onto outer wall **122** of seal member **120**, and perhaps instrument **"I".** As instrument **"I"** is advanced distally, instrument **"I"** deflects seal member **120** in a distal direction at angle **θ** formed with transverse axis **"B".** Thereafter, fluid **"F"** is communicated inwardly, across outer wall **122** of seal member **120,** thereby facilitating the lubrication of instrument "**I**" and seal member **120,** as discussed above with respect to each of the previous embodiments.

Upon the removal of instrument **"I",** the biasing force created by the biasing member (not shown) and exerted upon latch member **282** displaces latch member **282** radially inward, thereby returning latch member **282** to the first position, deactivating pump **280,** and arresting the communication of fluid **"F"** from reservoir.

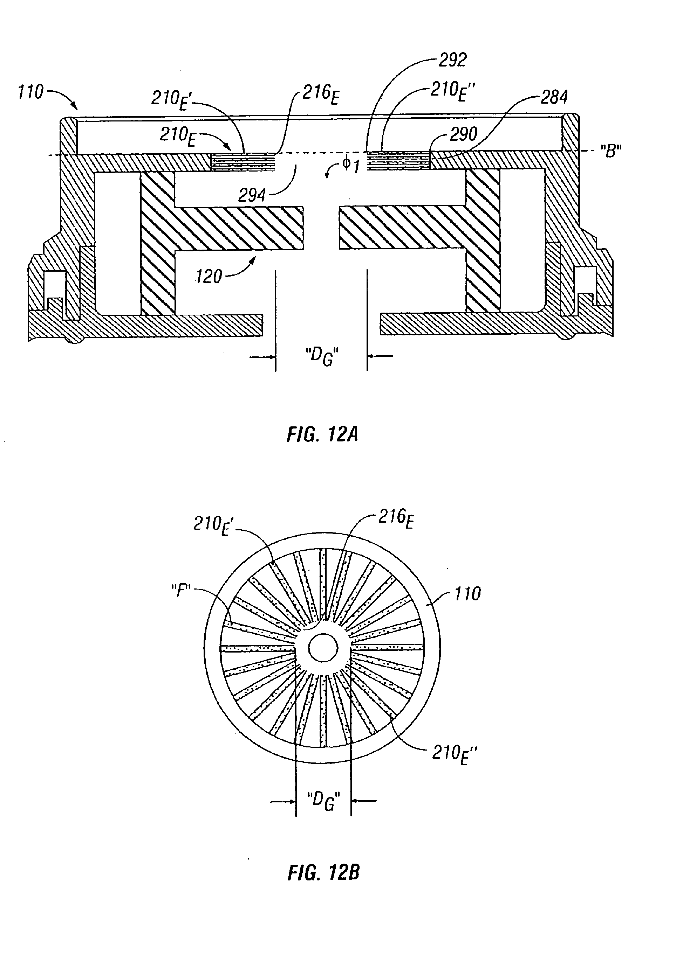

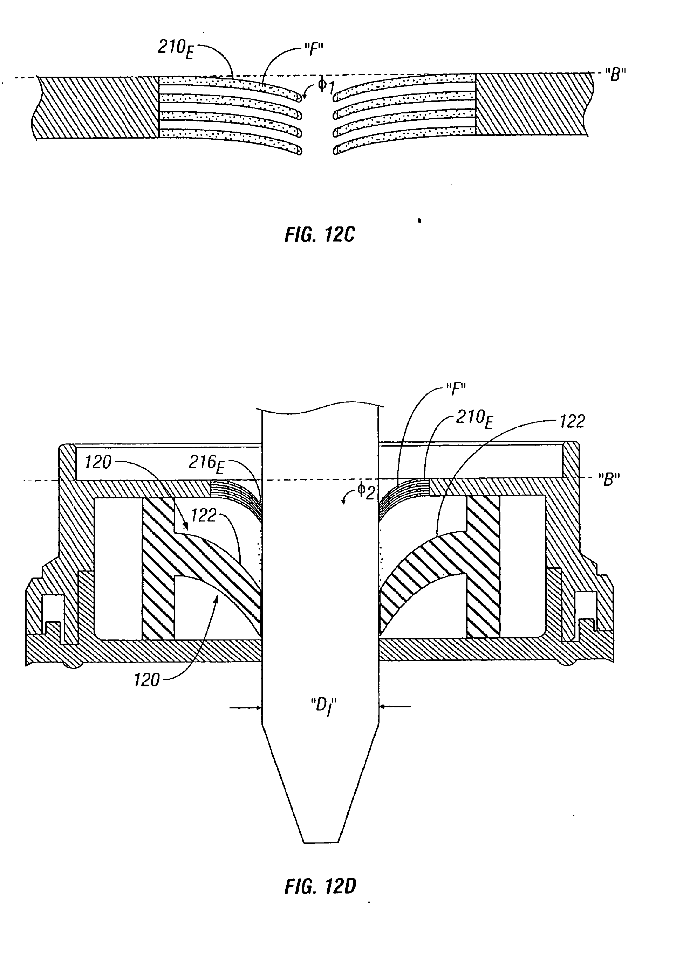

Referring now to **FIGS. 12A-12D**, in yet another embodiment, a plurality of vessels **210E**, which includes at least a first vessel **210_{E}**' and a second vessel **210_{E}**", are secured to inner wall **284** of housing **110** at first ends **290**. In this embodiment, vessels **210_{E}** are hollow, fingerlike structures that extend radially inward. The egress **216_{E}** of each vessel **210_{E}** is disposed at a second end **292** thereof and proximally of seal member **120.** Vessels **210_{E}** are configured such that an opening or gap **294** is defined between the adjacent second ends **292** of each pair of opposing vessels **210_{E}**. Opening **294** defines a diameter "**D_{G}**" that is appreciably lesser than the diameter "**D_{I}**" of surgical instrument **"I"** such that vessels **210_{E}** may engage instrument **"I"** upon the introduction thereof. The present disclosure contemplates that the opening **294** may be sufficiently dimensioned such that vessels **210_{E}** engage instrument **"I"** in sealing relation. Vessels **210_{E}** may be formed of any material that is at least partially resilient in nature such the vessels **210_{E}** may transition from a first condition (**FIGS. 12A-12C**) to a second condition **(****FIG. 12D****).**

In the first condition, vessels **210_{E}** are configured such that they define a first angle **θ₁** formed with axis **"B"**. Angle **θ₁** may be any angle that substantially prevents the discharge of fluid **"F"** from vessels **210_{E}** under the influence of the force of gravity and may include an angle of 0°, as seen in **FIG. 12A****,** or greater. Dependent upon the viscosity of fluid **"F",** in the first condition, vessels **210_{E}** may be oriented such that they exhibit a distal curvature, i.e. angle **θ₁** is greater than 0°, as seen in **FIG. 12C**. In the second condition, vessels **210_{E}** are configured such that they define a second angle **θ₂** formed with axis **"B".** Angle **θ₂** may be any angle that facilitates the discharge of fluid **"F"** from vessels **210_{E}** under the influence of the force of gravity, as seen in **FIG. 12D**. As fluid "**F**" is discharged from vessels **210_{E}** through egresses **216_{E}**, it is discharged onto instrument **"I"** and onto outer wall **122** of seal member **120** due to the proximal location of vessels **210_{E}** and egresses **216_{E}** in relation to seal member **120.**

As instrument "**I**" is advanced distally, instrument **"I"** engages and deflects seal member **120** in a distal direction. Thereafter, fluid "**F**" is communicated inwardly, across outer wall **122** of seal member **120**, thereby facilitating the lubrication of instrument **"I"** and seal member **120,** as discussed above with respect to each of the aforedescribed embodiments.

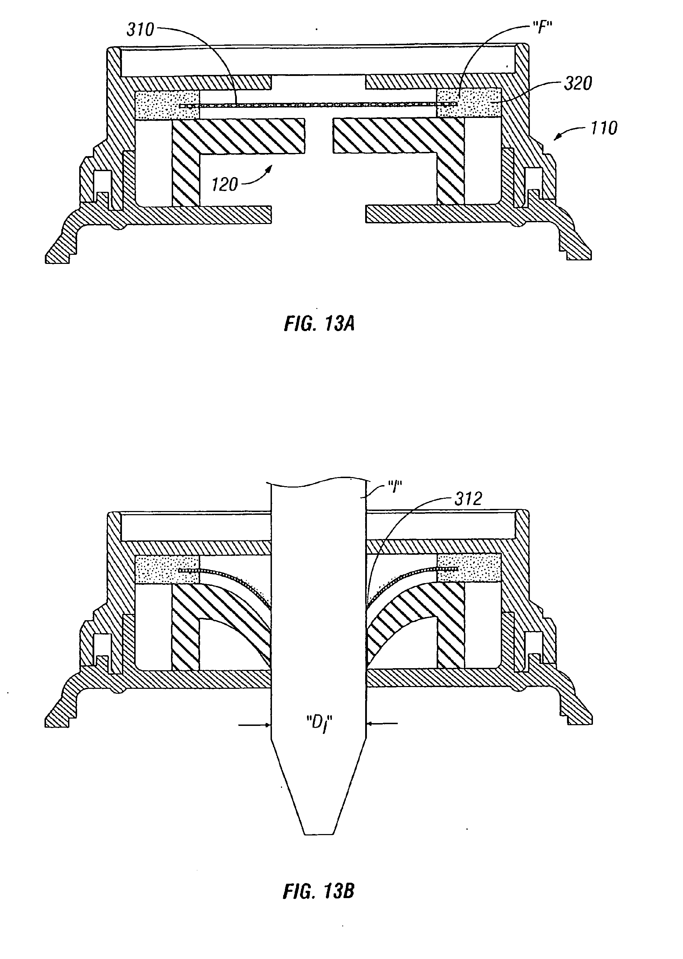

Referring now to **FIGS. 13A-13B**, another aspect of the present disclosure will be discussed in which the surgical access apparatus includes housing **110,** a wick member **310,** and seal member **120.**

In this aspect of the present disclosure, housing **110** includes at least one reservoir **320** disposed therein that is in fluid communication with wick member **310.** Reservoir **320** may be formed of individual structural elements, or may be simply defined by a recess or cavity formed within housing **110.**

Wick member **310** is any member that may be used to communicate or draw fluid **"F"** from reservoir **320** through capillary action, and accordingly, wick member **310** may be composed of any material suitable for that intended purpose including, but not limited to, natural fibers, such as cotton, or synthetic materials. Additionally, the material comprising wick member **310** may have characteristics that make wick member **310** at least semi-resilient, such that wick member **310** may deform upon the introduction of instrument "**I**", as discussed in further detail below.

Wick member **310** is disposed within housing **110** such that wick member **310** is located proximally of seal member **120**, and wick member **310** is at least partially disposed within the at least one reservoir **320** such that at least a portion of wick member **310** is disposed within fluid **"F".** It is contemplated that wick member **310** may be integrally formed with housing **110**, or that wick member **310** may be releasably formed therewith, thereby facilitating the replacement of wick member **310** when necessary.

Prior to the introduction of instrument **"I"**, wick member **310** is at least partially sodden with fluid "**F**", as seen in **FIG. 13A****.** Upon the introduction of surgical instrument **"I"** to wick member **310,** as seen in **FIG. 13B****,** fluid **"F"** is applied thereto. The proximal location of wick member **310** in relation to seal member **120** ensures that fluid "**F**" is applied to instrument "**I**" prior to the insertion of instrument **"I"** into seal member **120,** thereby facilitating the lubrication of instrument **"I"** and the longitudinal manipulation thereof within seal member **120,** as discussed above with respect to each of the aforementioned embodiments.

As instrument **"I"** is advanced distally, instrument **"I"** penetrates wick member **310**, thereby creating an opening, or a hole, **312** therein that substantially approximates the diameter "**D_{I}**" of instrument **"I".** The present disclosure contemplates that the opening **312** may be sufficiently dimensioned such that a seal is at least partially formed with instrument **"I".** The continued engagement of instrument **"I"** with wick member **310** resiliently enlarges the opening **312** in wick member **310** and ensures the substantially continuous application of fluid **"F"** to instrument **"I"**. In an alternate embodiment of wick member **310,** wick member **310** may define a pre-formed aperture (not shown) therein that is configured and dimensioned to receive instrument **"I"**, thereby obviating the need for puncture.

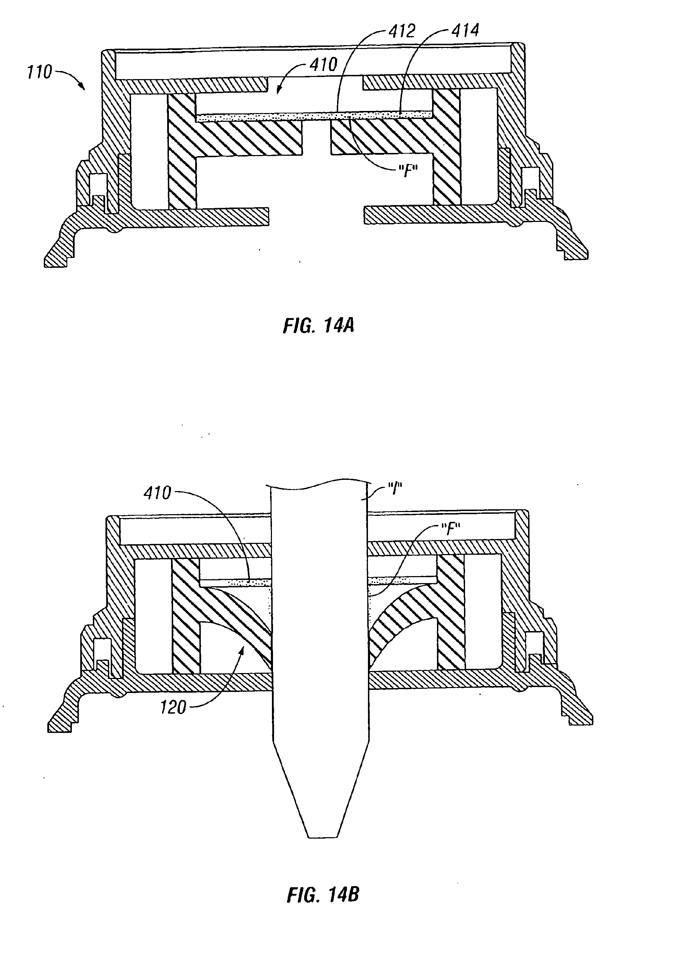

With respect to **FIGS. 14A-14B**, in yet another aspect of the present disclosure, the surgical access apparatus includes the housing **110,** a bladder member **410**, and seal member **120** disposed therein.

Bladder member **410** is disposed proximally of seal member **120,** and in one embodiment, bladder member **410** may sit directly atop seal member **120.** Bladder member **410** includes an outer wall **412** that defines an internal cavity **414** adapted to retain fluid "**F**". Bladder member **410** may be formed of any suitable material that is adapted for puncture by a surgical instrument **"I",** and may be either integrally formed with or releasably disposed within housing **110**, thereby facilitating the replacement thereof.

Upon the introduction of surgical instrument **"I"** to bladder member **410,** instrument **"I"** punctures outer wall **412,** thereby releasing the fluid **"F"** retained therein. As instrument **"I"** is advanced distally through bladder member **410,** fluid **"F"** is applied to instrument "**I**". The proximal location of bladder member **410** in relation to seal member **120** ensures that fluid **"F"** is applied to instrument **"I"** prior to the insertion of instrument **"I"** into seal member **120,** thereby facilitating the lubrication of instrument **"I"** and the longitudinal manipulation thereof within seal member **120,** as.discussed above with respect to each of the previous embodiments.

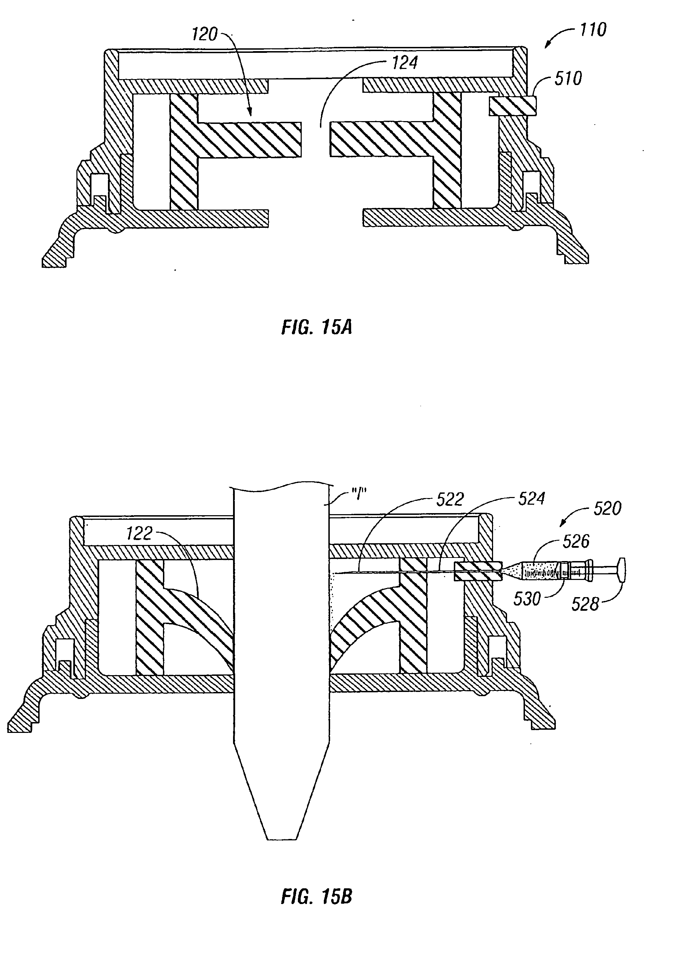

As seen in **FIGS. 15A-15B**, in a final aspect of the present disclosure, housing **110** includes a grommet member **510** that is disposed in an aperture **502** formed therein.

Grommet member **510** is disposed proximally of seal member **120** within housing **110** and is adapted for the insertion and removal of a needle member **520.** Grommet member **510** may be formed of any material suitable for this purpose, including but not limited to, polymeric materials. In one embodiment, grommet member **510** may define a pre-formed opening or passage (not shown) therethrough that is configured and dimensioned to receive needle member **520**. As depicted, needle member **520** includes a needle element **522** defining a channel **524** therethrough, a reservoir **526** having fluid "**F**" disposed therein, and a plunger element **528** disposed within reservoir **526** and coupled to a pusher **530.** It is contemplated that needle member **520** may be any member suitable for the intended purpose of retain and dispensing fluid **"F"**.

Upon the introduction of a surgical instrument **"I"** into housing **110,** needle member **520** is inserted through grommet member **510.** Subsequently, pusher **530** is advanced such that plunger element **528** may dispense fluid **"F"** through channel **524** in needle element **522.** Fluid **"F"** may be dispensed upon either or both of outer wall **122** or aperture **124** of seal member **120**. Alternatively, fluid **"F"** may be applied directly to instrument **"I"**. The proximal location of grommet member **510** in relation to seal member **120** ensures that fluid **"F"** is applied to instrument **"I",** either directly or through contact with seal member **120,** prior to the insertion of instrument **"I",** thereby facilitating the lubrication of instrument **"I"** and the longitudinal manipulation thereof within seal member **120,** as discussed above with respect to each of the aforementioned embodiments.

In each of the embodiments disclosed herein, it is contemplated that the surgical instrument **"I"** may itself be lubricated prior to its introduction to any of the aforedescribed housings, either manually by a clinician, or through the employ of a self-lubricating system associated with the instrument.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

The present disclosure relates to surgical access assemblies and surgical valves or seals of the type adapted to allow the introduction of a surgical instrument or object therethrough. In particular, the surgical access assemblies and seals disclosed herein are adapted to facilitate the insertion, withdrawal, and manipulation of a surgical instrument through the incorporation of a lubricous substance or fluid.
The invention may be described by reference to the following numbered paragraphs:
1. A surgical access apparatus comprising: housing; and a seal member disposed within the housing, the seal member including at least one reservoir adapted to accommodate a fluid and having at least one egress such that the fluid may be discharged from the at least one reservoir, wherein the at least one egress is configured and dimensioned to communicate the at least one fluid externally of the seal member.
2. The surgical access apparatus of paragraph 1, wherein the seal member is at least partially formed from a deformable material such that the seal member may transition from a first condition, in which the fluid is retained within the at least one reservoir, to a second condition, in which the fluid is discharged from the at least one reservoir.
3. The surgical access apparatus of paragraph 2, wherein the reservoir defines a fluid retaining capacity which decreases as the seal member transitions from the first condition to the second condition such that the fluid is expelled therefrom.
4. The surgical access apparatus of paragraph 2, wherein the seal member includes a veneer member secured to the periphery thereof for facilitating the retention of the fluid within the at least one reservoir.
5. The surgical access apparatus of paragraph 4, wherein the veneer member is adapted to be penetrated by a surgical instrument upon the insertion thereof into the housing.
6. The surgical access apparatus of paragraph 2, wherein the at least one reservoir includes a plurality of reservoirs that are substantially arcuate in configuration.
7. The surgical portal assembly of paragraph 6, wherein the plurality of reservoirs are concentrically disposed within the seal member.
8. A surgical access apparatus comprising: a housing having at least one vessel associated therewith, the at least one vessel being defined by a plurality of walls that describe an interior space that is configured and dimensioned to retain a fluid, the at least one vessel having at least one egress that is configured and dimensioned for fluid communication such that the fluid may be dispensed from the at least one vessel; a cannula sleeve extending from the cannula housing; and a seal member disposed within the housing and defining an aperture therethrough.
9. The surgical access apparatus of paragraph 8, wherein the at least vessel is mounted to an inner wall of the cannula housing, the at least one vessel being engagable with a surgical instrument upon insertion of the surgical instrument into the surgical access apparatus such that the fluid is discharged from the at least one vessel through the at least one egress.
10. The surgical access apparatus of paragraph 8, wherein the housing defines at least one channel therein configured and dimensioned to communicate a gas, the at least one channel being disposed distally of the at least one egress such that the fluid may be drawn out of the at least one vessel upon the communication of the gas.
11. The surgical access apparatus of paragraph 8 further including a pump member operatively associated with the at least one vessel.
12. The surgical access apparatus of paragraph 11 further including a latch disposed on an inner wall of the housing and operatively associated with the pump member, the latch member being movable from a first position to a second position upon the insertion of a surgical instrument into the internal cavity, wherein the latch member activates the pump member in the second position.
13. The surgical access apparatus of paragraph 8, wherein the seal member is movable from a first position to a second position upon the insertion of a surgical instrument into the aperture thereof, the seal member substantially abutting the at least one egress in the first position and at least partially exposing the at least one egress in the second position.
14. The surgical access apparatus of paragraph 13 further including a biasing member disposed within the at least one vessel to facilitate dispensation of the fluid.
15. The surgical access apparatus of paragraph 8, wherein the at least one vessel further includes at least one ingress in fluid communication with the interior space defined by the plurality of walls of the at least one vessel through a channel defined in the cannula sleeve.
16. The surgical access apparatus of paragraph 15, wherein the ingress is disposed distally of the at least one egress.
17. The surgical access apparatus of paragraph 16 further including a one-way valve disposed within the channel and adjacent the at least one ingress, wherein the one way valve is configured and dimensioned such that the fluid retained within the at least one vessel is substantially prevented from exiting the channel through the at least one ingress.
18. A surgical access apparatus comprising: a housing having at least one reservoir disposed therein, the at least one vessel being adapted to retain a fluid therein; a seal member disposed within the cannula housing; and a wick member in fluid communication with the at least one reservoir, the wick member being at least partially disposed within the housing and proximally of the seal member.
19. A surgical access apparatus comprising: a cannula housing; a seal member disposed within the cannula housing; and a bladder member disposed within the cannula housing, the bladder member defining an internal cavity adapted to retain a fluid therein, the fluid being exuded from the bladder member upon puncture by a surgical instrument.
20. A surgical access apparatus comprising: a cannula housing; a seal member disposed within the cannula housing; and a grommet member associated with the cannula housing, the grommet member being adapted for penetration by a needle member that is configured and dimensioned to communicate a fluid therethrough, the grommet member being oriented such that the fluid may be dispensed about the seal member.

## Claims

1. A surgical access apparatus 100 comprising:
a housing 110 having at least one vessel 210 associated therewith, the at least one vessel being defined by a plurality of walls 212 that describe an interior space 214 that is configured and dimensioned to retain a fluid, the at least one vessel 210 having at least one egress 216 that is configured and dimensioned for fluid communication such that the fluid may be dispensed from the at least one vessel 210, and a biasing member 218 disposed within the at least one vessel 210 to facilitate dispensation of the fluid, the biasing member 218 including a pusher element 220 that is operatively connected to a spring 222;
a cannula sleeve 130 extending from the housing;
a seal member 120 disposed within the housing and defining an aperture therethrough; and
**characterised in that** the seal member 120 is movable from a first position to a second position upon the insertion of a surgical instrument into the aperture thereof, the seal member substantially abutting the at least one egress 216 in the first position and at least partially exposing the at least one egress in the second position.

2. The surgical access apparatus of claim 1, wherein the egress 216 is disposed proximally of seal member 210.

3. The surgical access apparatus of claim 1, wherein the vessel 210 includes a channel 224 that extends proximally therefrom and terminates in an opening 226 in housing 110.

4. The surgical access apparatus of claim 1, wherein the housing 110 includes a conduit 260 defined therein that is in fluid communication with an inlet port 270 formed in housing 210.

5. The surgical access apparatus of claim 4, wherein the conduit 260 extends through seal member 120 and is disposed distally of egress 216c formed in vessel 210c.

6. The surgical access apparatus of claim 5, wherein the conduit 260 is configured to communicate an insufflation gas 140 therethrough.
